# EUROPEAN PATENT APPLICATION

(11) **EP 4 585 056 A1**
(43) Date of publication of application: **16.07.2025**
(21) Application number: 24212976.5
(22) Date of filing: 14.11.2024
(51) Int. Cl.: A23L 33/135, A61K 35/74, A61P 19/08, A61P 19/10, A61P 21/00

(54) **COMPOSITION FOR PREVENTING OR TREATING BONE AND MUSCLE DISEASE CONTAINING EUBACTERIUM NODATUM**

(30) Priority: 09.01.2024 KR 20240003360
(71) Applicant: Korea Institute of Science and Technology, Seoul 02792 (KR)
(72) Inventor: KIM, MYUNG SUK, 25451 Gangwon-do (KR); YOO, GY HYE, 25451 Gangwon-do (KR); LEE, CHOONG GU, 25451 Gangwon-do (KR); PARK, YOUNG TAE, 25451 Gangwon-do (KR); CHA, KWANG HYUN, 25451 Gangwon-do (KR); HONG, SOYEON, 25451 Gangwon-do (KR); NGUYEN, BAO NGOC, 25451 Gangwon-do (KR)
(74) Representative: Handley, Matthew Edward

(57) **Abstract**

The present invention relates to a composition for preventing or treating bone and muscle disease comprising *Eubacterium nodatum* or a culture thereof. The present composition comprising *Eubacterium nodatum* or a culture thereof have the effect of increasing bone density, bone area, bone volume and bone thickness, increasing grip strength and muscle weight, and improving athletic performance.

## Description

### [Technical field]

This application claims benefit of priority based on Korean Patent Application No. 10-2024-0003360 filed on January 9, 2024, the disclosures of which are incorporated herein by reference in their entireties.

The present invention relates to a composition for preventing or treating bone and muscle disease comprising *Eubacterium nodatum.*

### [Background art]

Muscle can be categorized into skeletal muscle, cardiac muscle, and visceral muscle, with skeletal muscle being the most abundant tissue in the human body, making up 40-45% of body weight. Skeletal muscles attach to bones via tendons and are responsible for generating bone movement or force. A muscle is made up of many myofibrils, which in turn are made up of many fibrils, which are made up of actin and myosin. When actin and myosin overlap and move, the length of the muscle shortens or lengthens, causing contraction and relaxation of the overall muscle. An increase in the size of the myofibrils means an increase in the thickness of the myofibers, resulting in muscle gain.

Muscle diseases are characterized by a progressive progression of skeletal muscle weakness that leads to impaired walking and mobility, difficulty with activities of daily living (ADLs), and loss of independence. In addition, they can lead to cardiopulmonary dysfunction and other complications, so it is important to accurately understand the characteristics of each muscle disease and approach it accordingly.

South Korea entered an aging society in 2000 with the elderly population accounting for 7.2% of the total population, and is expected to enter a super-aging society (more than 20%) in 2050 (2013 statistics on the elderly, Statistics Korea). As a person's muscle mass decreases with age (by 10-15% between the ages of 50 and 70, and by more than 30% between the ages of 70 and 80), grip strength and muscle function also decrease, a condition known as sarcopenia. Senile sarcopenia is a major cause that limits the independent living of older people by causing activity and walking difficulties. In addition, sarcopenia lowers basal metabolic rate, increases insulin resistance, promotes type 2 diabetes, and increases the risk of high blood pressure and cardiovascular disease by 3-5 times. Currently, there are no drugs approved for the treatment of sarcopenia, and drug repositioning technology that applies myostatin inhibitors or existing FDA-approved treatments for other diseases to sarcopenia is being developed.

Osteoporosis, a typical example of a bone disease, is a condition in which the balance between bone formation and bone resorption is disrupted, resulting in a loss of bone density. In the United States alone, approximately 10 million people already have osteoporosis and 18 million are at risk of developing osteoporosis. In South Korea, approximately 4 million people have or are at risk of osteoporosis. This number is expected to increase as we enter an aging society, resulting in increased societal expenditures and the mental and economic burden on family members.

As a result of efforts to develop novel materials effective in preventing or treating bone and muscle diseases, the present inventors completed the present invention by experimentally confirming that *Eubacterium nodatum* is useful for increasing bone density, bone volume, and bone thickness, and for improving muscle strength and athletic performance.

### [Disclosure]

### [Technical Problem]

It is an object of the present invention to provide a pharmaceutical composition for preventing or treating bone and/or muscle disease, or improving athletic performance, comprising *Eubacterium nodatum* as an active ingredient.

It is also an object of the present invention to provide a food composition for preventing or ameliorating bone and/or muscle disease, or improving athletic performance, comprising *Eubacterium nodatum* as an active ingredient.

It is also an object of the present invention to provide a quasi-drug composition for preventing or ameliorating bone and/or muscle disease, or improving athletic performance, comprising *Eubacterium nodatum* as an active ingredient.

It is also an object of the present invention to provide a method for preventing or treating bone and/or muscle disease, or a method for improving athletic performance (or exercise capacity), comprising administering or taking a composition comprising *Eubacterium nodatum* or a culture thereof as an active ingredient to an individual in need.

The present invention also provides a food, quasi-drug or pharmaceutical composition for use in preventing or treating bone and/or muscle disease or improving athletic performance, comprising a composition comprising *Eubacterium nodatum* or a culture thereof as an active ingredient.

However, the technical problem to be achieved by the present invention is not limited to the problems mentioned above, and other problems not mentioned will be clearly understood by those skilled in the art from the description below.

### [Technical solution]

The present invention provides a food composition for preventing or ameliorating bone and/or muscle disorders comprising *Eubacterium nodatum* or a culture thereof as an active ingredient.

The present invention also provides a quasi-drug composition for preventing or ameliorating bone and/or muscle disease, or improving athletic performance, comprising *Eubacterium nodatum* or a culture thereof as an active ingredient.

The present invention also provides a pharmaceutical composition for preventing or treating bone and/or muscle disease, or improving athletic performance, comprising *Eubacterium nodatum* or a culture thereof as an active ingredient.

The present invention also provides a method for preventing or treating bone and/or muscle disease, or a method for improving athletic performance (or exercise capacity), comprising administering or taking a composition (or a pharmaceutical composition) comprising *Eubacterium nodatum* or a culture thereof as an active ingredient to an individual in need.

The present invention also provides a food, quasi-drug or pharmaceutical composition for use in preventing or treating bone and/or muscle disease, or improving athletic performance, comprising a composition comprising *Eubacterium nodatum* or a culture thereof as an active ingredient.

The muscle disease may be one or more diseases selected from the group consisting of sarcopenia, muscular atrophy, myasthenia, muscular dystrophy, myotonia, hypotonia, muscular weakness, muscular dystrophy, atony, amyotrophic lateral sclerosis, and inflammatory myopathy.

The bone disease may be one or more diseases selected from the group consisting of osteoporosis, osteomalacia, osteopenia, bone atrophy, fibrous dysplasia, Paget's disease, hypercalcemia, neoplastic bone destruction, and growth disorder of the bone.

The composition may improve grip strength and/or muscle strength.

The composition may increase muscle weight.

The composition may increase bone density, bone area, bone volume, and/or bone thickness.

The composition may be formulated as a capsule, tablet, powder, or beverage.

The dosages(or amount) of *Eubacterium nodatum* or a culture thereof as an active ingredient in the composition may be in the range of 0.001-100 mg/kg body weight per day.

The *Eubacterium nodatum* may be a live strain.

The *Eubacterium nodatum* may be accession number ATCC No. 33099 strain.

### [Advantageous Effects]

*The Eubacterium nodatum* of the present invention has the effect of increasing bone density, bone volume, and bone thickness. In addition, *The Eubacterium nodatum* of the present invention has the effect of increasing grip strength, increasing muscle weight, and improving athletic performance. Furthermore, the present composition comprising *Eubacterium nodatum* as an active ingredient can be applied to various products such as pharmaceutical composition, food composition, and quasi-drug composition for the prevention, treatment or improvement of bone and muscle diseases.

### [Description of Drawings]

Figure 1 is a schematic diagram of the design of an animal model to test the effectiveness of *Eubacterium nodatum* in treating or ameliorating osteopenia.
Figure 2 shows the effects of *Eubacterium nodatum* on bone mineral density, bone area, bone volume, and bone thickness (129S1: 129S1/Svlmj mice, Control: Control, Dex: Dexamethasone, Ab: Antibiotics, EN: *Eubacterium nodatum*; *, p < 0.05; ** p < 0.01; ***, p < 0.001).
Figure 3 shows the results of *Eubacterium nodatum*'s effect on grip strength and weight gain by muscle type (129S1: 129S1/Svlmj mice, Control: Control, Dex: Dexamethasone, Ab: Antibiotics, EN: *Eubacterium nodatum,* BW: body weight; *, p < 0.05; ** p < 0.01; ***, p < 0.001).
Figure 4 shows the results of *Eubacterium* nodatum's effect on improving exercise performance (B6: C57BL/6J mice, Control: Control, Dex: Dexamethasone, Ab: Antibiotics, *EN: Eubacterium nodatum*; *, p < 0.05; ** p < 0.01; ***, p < 0.001).

### [Mode of the Invention]

The present inventors completed the present invention by experimentally confirming that a strain of *Eubacterium nodatum* with few or no side effects is effective in increasing bone mineral density, bone volume and bone thickness, and improving grip strength and athletic performance.

Hereinafter, the present invention will be described in detail.

The present invention provides a composition for preventing, ameliorating or treating bone and/or muscle disease comprising *Eubacterium nodatum* or a culture thereof as an active ingredient.

The strain may be a live strain or an attenuated strain (such as, killed strain). "Attenuation" means modifying the strain to reduce its pathogenicity. Attenuation can be intended to reduce toxicity and other adverse effects when the strain is administered to a subject. Attenuated strains can be produced by a variety of methods known in the art. For example, attenuation can be achieved by deleting or disrupting virulence factors that allow the strain to survive in the host cell. Such deletion or disruption is well known in the art and can be accomplished by methods such as, for example, homologous recombination, chemical mutagenesis, irradiation mutagenesis, or transposon mutagenesis.

The culture may be prepared by any combination of incubation conditions for a period of time or longer from which an effective active ingredient/substance can be released. Preferably, it may be incubated for at least 24 hours, at least 48 hours or at least 72 hours, more preferably for at least 48 hours, but not limited to.

The muscle disease can be one or more diseases selected from the group consisting of sarcopenia, muscular atrophy, myasthenia, muscular dystrophy, myotonia, hypotonia, muscular weakness, muscular dystrophy, atony, amyotrophic lateral sclerosis, and inflammatory myopathy.

The bone disease can be one or more diseases selected from the group consisting of osteoporosis, osteomalacia, osteopenia, bone atrophy, fibrous dysplasia, Paget's disease, hypercalcemia, neoplastic bone destruction, and growth disorder of the bone.

The composition can enhance grip strength.

The composition can increase athletic performance.

The composition can increase muscle weight.

The composition can increase bone density, bone area, bone volume, and/or bone thickness.

The composition may be a food composition, a dietary supplement composition, a pharmaceutical composition, or a quasi-drug composition.

As used herein, "preventing" means any act of delaying the onset of a muscle disease by administration of a composition of the invention, and "treating" and "ameliorating/ improving" means any act of ameliorating or beneficially altering the symptoms of a muscle disease by administration of a composition of the invention.

As used herein, the term 'comprising as an active ingredient' means containing a sufficient amount to achieve the efficacy or activity of *Eubacterium nodatum.* The upper limit of the quantitative amount of *Eubacterium nodatum* included in a composition of the present invention may be selected and practiced by those skilled in the art within any suitable range.

### Pharmaceutical composition for preventing or treating bone and/or muscle disease comprising Eubacterium nodatum as an active ingredient

The composition of the present invention can be formulated as pharmaceutical composition.

When the composition of the present invention is formulated as a pharmaceutical composition, the pharmaceutical composition of the present invention may comprise a pharmaceutically acceptable carrier.

According to a preferred embodiment of the present invention, the composition of the present invention may be a pharmaceutical composition comprising (a) a pharmaceutically effective amount of *Eubacterium nodatum* of the present invention as described above; and (b) a pharmaceutically acceptable carrier. As used herein, the term "pharmaceutically effective amount" refers to an amount sufficient to achieve the efficacy or activity of *Eubacterium nodatum* described above.

Pharmaceutically acceptable carriers are those customarily used in the formulation: lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oils, but not limited thereto. In addition to the above ingredients, the pharmaceutical composition of the present invention may further include lubricants, wetting agents, sweeteners, flavoring agents, emulsifiers, suspending agents, preservatives, etc. Suitable pharmaceutically acceptable carriers and formulations are described in detail in Remington's Pharmaceutical Sciences (19th ed., 1995).

The pharmaceutical composition of the present invention can be administered orally or parenterally.

Suitable dosages of the pharmaceutical composition of the present invention can be prescribed in a variety of ways, depending on factors such as the method of formulation, mode of administration, patient age, weight, sex, medical condition, food, time of administration, route of administration, rate of excretion, and response sensitivity. Typical dosages of the active ingredients in the pharmaceutical composition of the present invention are in the range of 0.001-100 mg/kg/day, preferably 0.01-35 mg/kg/day in adults. The dose may be administered once daily or may be divided into several doses. However, the above dosages do not limit the scope of the present invention.

The pharmaceutical composition of the present invention may be prepared in unit dose form or by formulation with pharmaceutically acceptable carriers and/or excipients, in accordance with methods readily practiced by a person of ordinary skill in the art to which the invention belongs. The formulation may be in the form of a solution, suspension, syrup, or emulsion in an oil or aqueous medium, or in the form of an excipient, acid, powder, granule, tablet, or capsule, and may further comprise a dispersant or stabilizing agent.

### Quasi-drug composition for preventing or ameliorating bone and/or muscle disorders comprising Eubacterium nodatum as an active ingredient

The composition of the present invention can be provided as a prodrug composition.

*Eubacterium nodatum* may be added as is, or may be used together with ingredients such as other quasi-drugs, and may be used appropriately according to conventional methods. The mixing amount of the active ingredient can be appropriately determined depending on the purpose of use (prevention, health, or therapeutic treatment). The quasi-drug composition may be used in the manufacture of external preparations, patches, ointments, etc., but is not limited thereto.

### Food composition for preventing or ameliorating bone and/or muscle disease comprising Eubacterium nodatum as an active ingredient

The composition of the invention may be provided as food composition or dietary supplement composition. When the composition for preventing, ameliorating or treating bone and/or muscle disease comprising *Eubacterium nodatum* of the present invention as an active ingredient is manufactured as a food composition, it may include not only *Eubacterium nodatum* of the present invention, but also ingredients that are customarily added in the manufacture of food products, for example, proteins, carbohydrates, fats, nutrients, seasonings and flavorings. Examples of the carbohydrates described above are monosaccharides, e.g., glucose, fructose, and the like; disaccharides, e.g., maltose, sucrose, oligosaccharides, and the like; and polysaccharides, e.g., dextrins, cyclodextrins, and the like, and sugar alcohols, e.g., xylitol, sorbitol, erythritol, and the like. As flavoring agents, natural flavoring agents [such as thaumatin, stevia extract (e.g., rebaudioside A, glycyrrhizin, etc.)] and synthetic flavoring agents (such as saccharin, aspartame, etc.) can be used. For example, if the food composition of the present invention is formulated as a beverage, it may additionally contain citric acid, liquid dextrose, sugar, glucose, acetic acid, malic acid, fruit juice, caterpillar extract, jujube extract, licorice extract, etc. in addition to the natural product extract of the present invention.

The formulation of the food composition or dietary supplement composition may be in the form of an acid, granule, pill, tablet, capsule, or capsule, as well as in the form of a conventional food or beverage.

Examples of food products to which the substance may be added include, without limitation, meat, sausages, bread, chocolate, candy, snacks, confectionery, pizza, ramen, other noodles, chewing gum, dairy products including ice cream, various soups, beverages, teas, drinks, alcoholic beverages and vitamin complexes, all of which are food products in the ordinary sense of the term.

In general, when manufacturing food or beverages, *Eubacterium nodatum* can be added in an amount of 15 parts by weight or less, preferably 10 parts by weight or less, based on 100 parts by weight of raw materials. However, in the case of long-term intake for the purpose of health and hygiene or health control, the amount may be below the above range, and the present invention may also be used in amounts above the above range because there is no problem in terms of safety.

Hereinafter, the present invention will be described in more detail through examples. The purpose, features, and advantages of the present invention will be easily understood through the following examples. The present invention is not limited to the embodiments described herein and may be embodied in other forms. The embodiments introduced here are provided to enable the idea of the present invention to be sufficiently conveyed to those skilled in the art. Therefore, the present invention should not be limited by the following examples.

### [Example 1] Isolation of Eubacterium nodatum strain, preparation of culture thereof and analysis of bone health-related markers

*Eubacterium nodatum* (ATCC No. 33099), obtained from the American Type Culture Collection (ATCC), was liquid cultured under anaerobic conditions in Reinforced Clostridial Medium (RCM) medium for 3 days, filtered to prepare microbial cultures, which were then analyzed for bone health-related markers such as bone density using control mice and a mouse model of osteopenia.

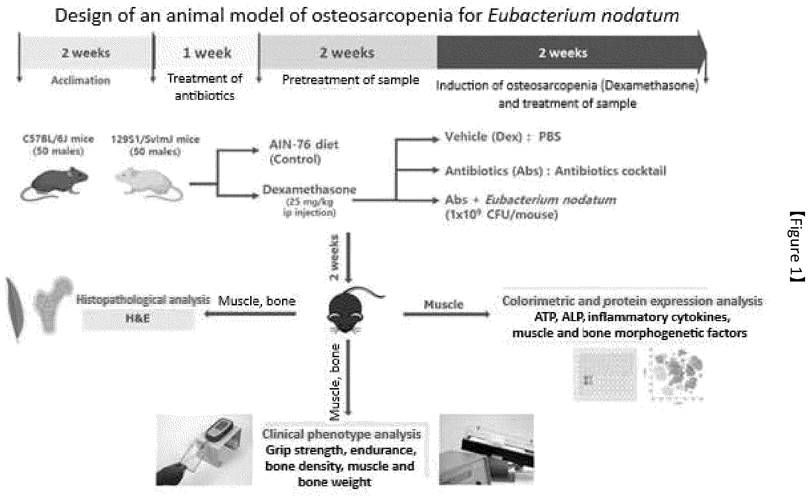

The mice used to test the efficacy of *Eubacterium nodatum* were prepared as described below. As shown in Figure 1, C57BL/6J mice (male, 12 weeks old) and 129S1/Svlmj mice (male, 12 weeks old) were obtained from Dooyeol Biotech, and after a 2-week acclimatization period, they were divided into 4 groups (n=10 per group) according to body weight. The diets, doses, and methods of administration of the experimental groups were shown in Table 1 below. Mice in two of the four groups were fed the antibiotic diet for 1 week. The test substance, *Eubacterium nodatum,* was prepared as a liquid suspension in PBS and administered for 4 weeks. The vehicle control group was orally administered PBS daily to compensate for the placebo effect of PBS and the weight loss caused by the stress of administration. Two weeks after administration of test substance, the experimental group received an intraperitoneal injection of dexamethasone for 2 weeks to induce osteopenia, while the normal control group received an intraperitoneal injection of saline. All mice were fed the same AIN76 diet (Research Diets) and treated with the test substance for an additional 2 weeks for a total of 4 weeks. Dark : Light cycles were maintained at 12 hr : 12 hr intervals and water was provided ad libitum.

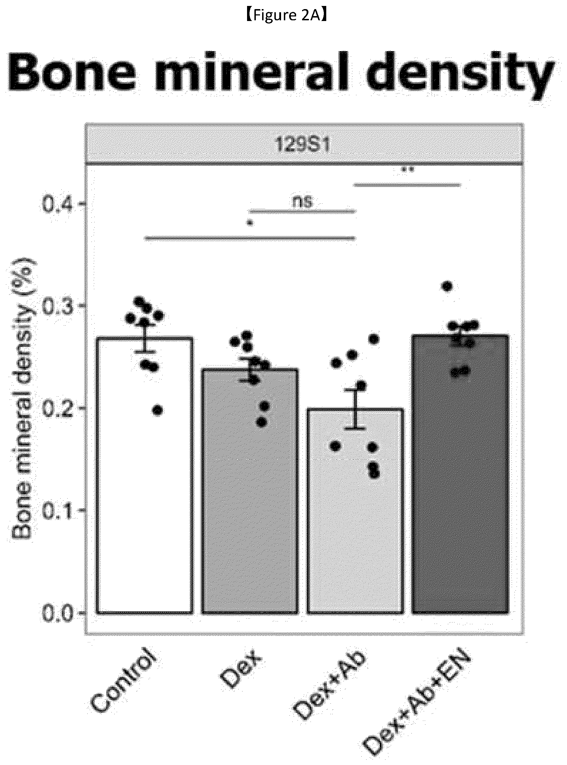

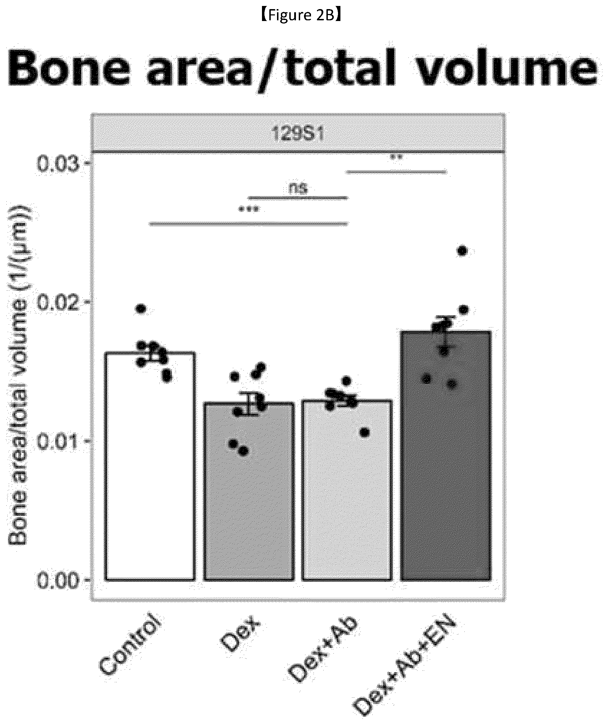

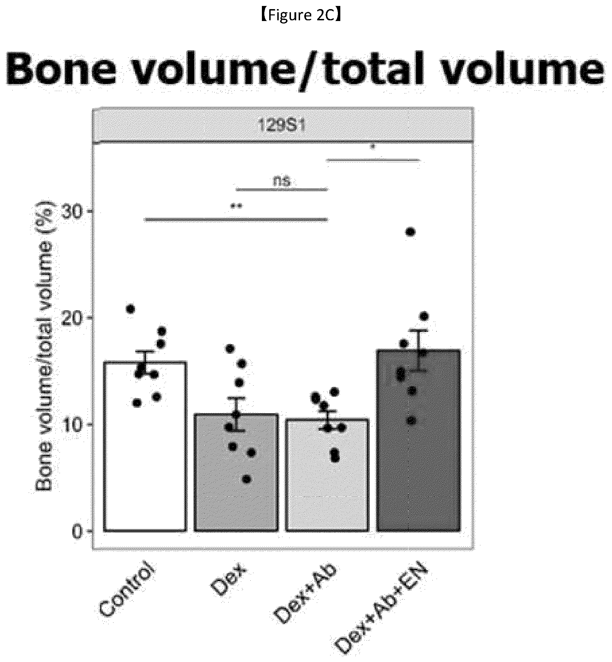

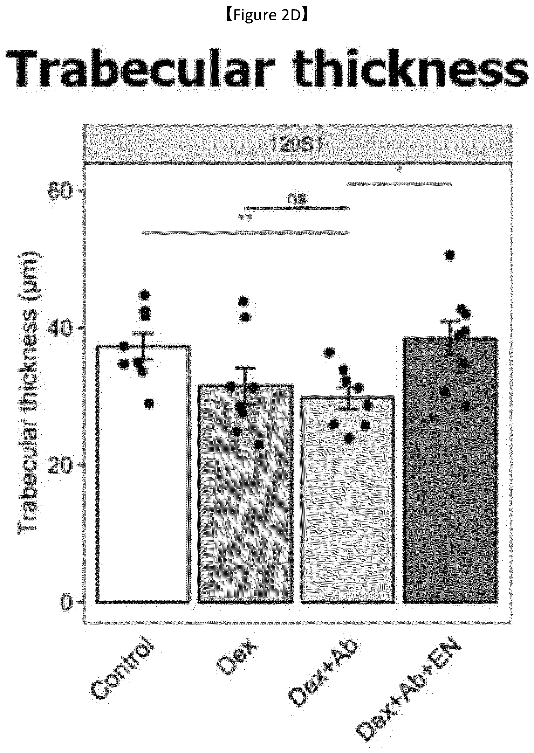

The experimental results showed that bone mineral density, bone area, bone volume and bone mineral thickness decreased in the Dexamethasone (Dex) group as shown in Figure 2, and it was confirmed that bone mineral density, bone area, bone volume and bone thickness increased again upon administration of *Eubacterium nodatum* (Dex: Dexamethasone osteopenia inducing substance (25 mg/kg), Ab: Antibiotics (Ampicillin 1 g/L, Metronidazole 1 g/L, Neomycin trisulfate 1 g/L, Vancomycin 0.5g/L), EN: *Eubacterium nodatum* (1 x 10⁹ CFU/mouse)).

**[Table 1]**

| Test group | Feed | Osteopeniainducing substance | Orally administered substance | Dosage and method of administrati on | Administration period |
|---|---|---|---|---|---|
| Control | AIN76 diet | Saline | Vehicle | - | After 2 weeks of oral administration of test substance, 2 weeks of administration of test substance and inducing substance (Total 4 weeks) |
| Dex | | Dexamethasone (25 mg/kg, once daily, for 2 weeks) | Vehicle | - | |
| Dex+Ab | | | Antibiotics (1 week) | Supplied mixed with drinking water¹⁾ | |
| Dex+Ab+EN | | | Antibiotics (1 week) | Supplied mixed with drinking water¹⁾ | |
| | | | *Eubacterium nodatum (2* weeks) | 1 × 10⁹ CFU/mouse Once per day | |

| | | | | | |
|---|---|---|---|---|---|
| ¹⁾ Antibiotic cocktail (Ampicillin 1g/L, Metronidazole 1g/L, Neomycin trisulfate 1g/L, Vancomycin 0.5g/L) mixed in drinking water for 1 week | | | | | |

### [Example 2] Confirmation of the effect of improving grip strength in mice with sarcopenia induced by dexamethasone administration

The same animal model (129S1/Svlmj mice) as in Example 1 above was used to confirm the effect of *Eubacterium nodatum* on grip strength.

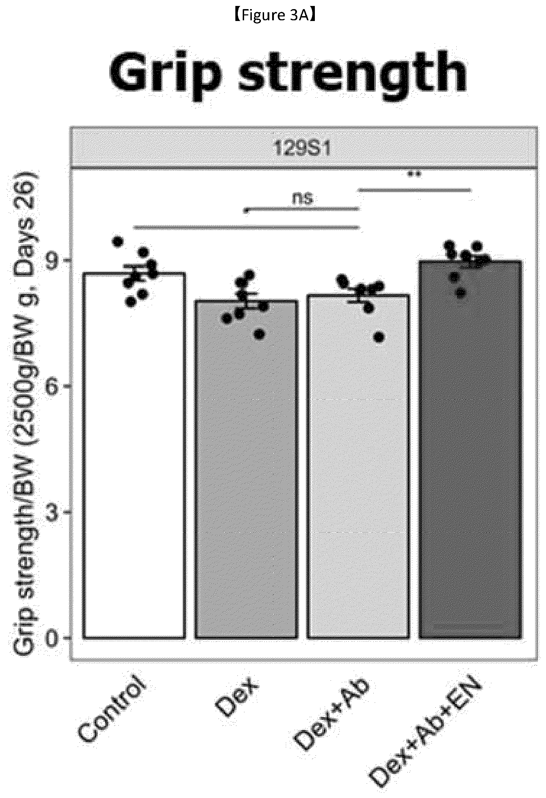

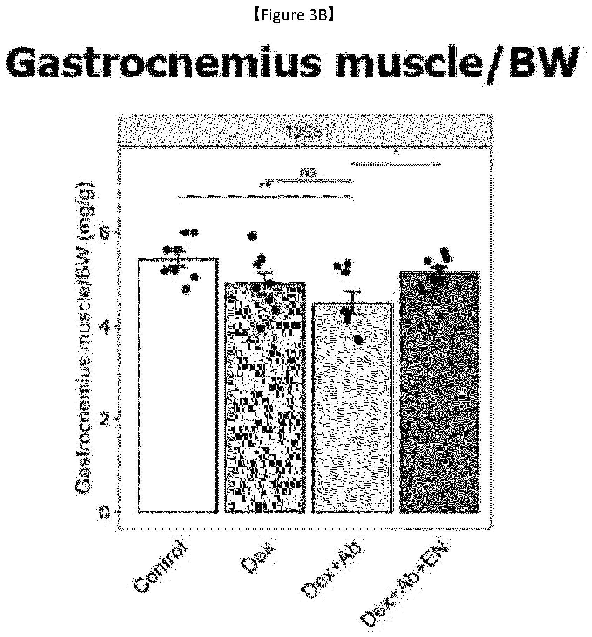

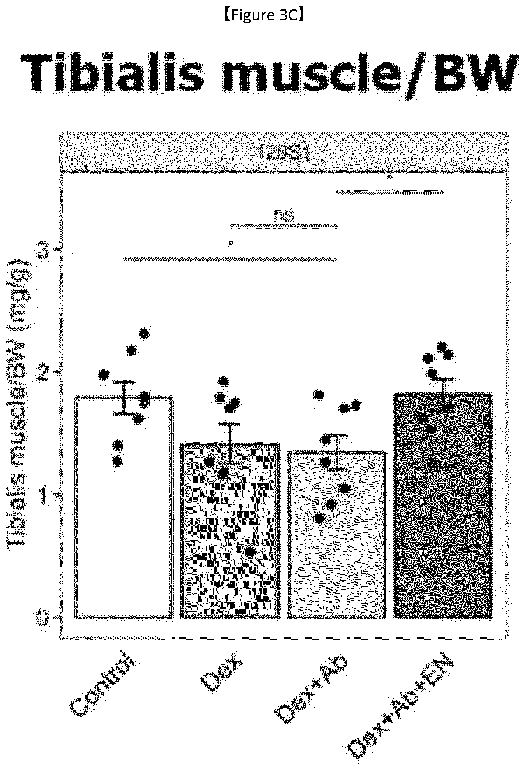

As shown in Figure 3, it was confirmed that the grip strength decreased in the Dexamethasone (Dex)-treated group and increased again by the administration of *Eubacterium nodatum.*

### [Example 3] Confirmation of weight increase by muscle type in mice with osteosarcoma induced by dexamethasone administration

The same animal model (129S1/Svlmj mice) as in Example 1 above was used to confirm the effect of *Eubacterium nodatum* on muscle weight gain.

As shown in Figure 3, compared to the control group administered saline, the dexamethasone-treated group was found to have a decrease in the vastus lateralis and calf muscles, but a significant increase in the weight of these muscles was found upon administration of *Eubacterium nodatum.*

### [Example 4] Confirmation of efficacy in improving exercise performance ability

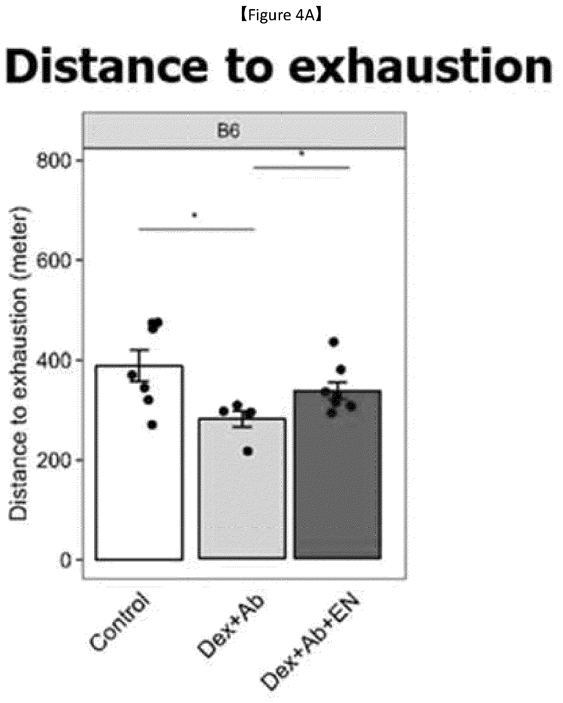

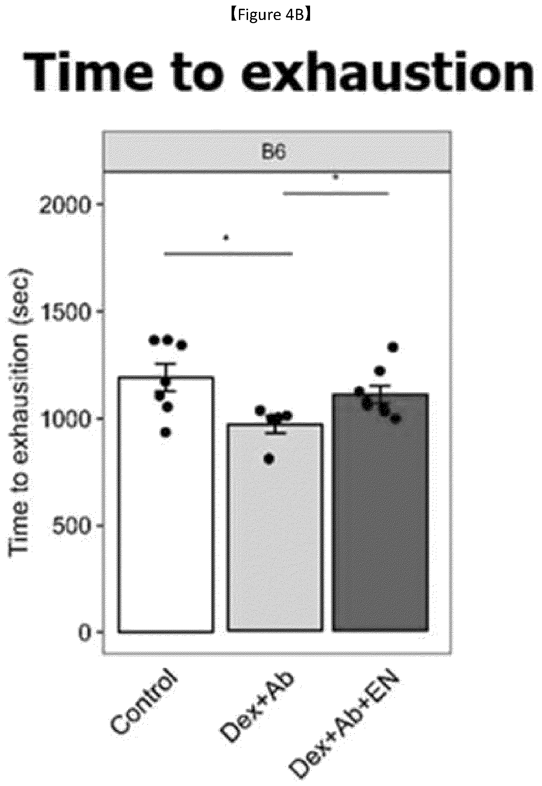

The same animal model (C57BL/6J mice) as in Example 1 above was used to evaluate the exercise performance induced by *Eubacterium nodatum.* Specifically, maximum running speed, distance to exhaustion, and time to exhaustion were evaluated and the results were shown in Figure 4. As shown in Figure 4, it was found that the exercise performance decreased by antibiotic administration was significantly increased by the administration of *Eubacterium nodatum.*

## Claims

1. A food composition for use in preventing or ameliorating bone and muscle disease, or bone disease or muscle disease, or improving athletic performance, comprising *Eubacterium nodatum* or a culture thereof as an active ingredient.

2. The food composition for use of claim 1, wherein the bone disease is one or more diseases selected from the group consisting of osteoporosis, osteomalacia, osteopenia, bone atrophy, fibrous dysplasia, Paget's disease, hypercalcemia, neoplastic bone destruction, and growth disorder of the bone.

3. The food composition for use of claim 1, wherein the muscle disease is one or more diseases selected from the group consisting of sarcopenia, muscular atrophy, myasthenia, muscular dystrophy, myotonia, hypotonia, muscular weakness, muscular dystrophy, atony, amyotrophic lateral sclerosis, and inflammatory myopathy.

4. The food composition for use of claim 1, wherein the composition increases a grip strength.

5. The food composition for use of claim 1, wherein the composition increases muscle weight.

6. The food composition for use of claim 1, wherein the composition has the effect of increasing bone density, bone area, bone volume, or bone thickness.

7. A quasi-drug composition for use in preventing or ameliorating bone and muscle disease, or bone disease or muscle disease, or improving athletic performance, comprising *Eubacterium nodatum* or a culture thereof as an active ingredient.

8. The quasi-drug composition for use of claim 7, wherein the bone disease is one or more diseases selected from the group consisting of osteoporosis, osteomalacia, osteopenia, bone atrophy, fibrous dysplasia, Paget's disease, hypercalcemia, neoplastic bone destruction, and growth disorder of the bone.

9. The quasi-drug composition for use of claim 7, wherein the muscle disease is one or more diseases selected from the group consisting of sarcopenia, muscular atrophy, myasthenia, muscular dystrophy, myotonia, hypotonia, muscular weakness, muscular dystrophy, atony, amyotrophic lateral sclerosis, and inflammatory myopathy.

10. A pharmaceutical composition for use in preventing or treating bone and muscle disease, or bone disease or muscle disease, or improving athletic performance, comprising *Eubacterium nodatum* or a culture thereof as an active ingredient.

11. The pharmaceutical composition for use of claim 10, wherein the bone disease is one or more diseases selected from the group consisting of osteoporosis, osteomalacia, osteopenia, bone atrophy, fibrous dysplasia, Paget's disease, hypercalcemia, neoplastic bone destruction, and growth disorder of the bone.

12. The pharmaceutical composition for use of claim 10, wherein the muscle disease is one or more diseases selected from the group consisting of sarcopenia, muscular atrophy, myasthenia, muscular dystrophy, myotonia, hypotonia, muscular weakness, muscular dystrophy, atony, amyotrophic lateral sclerosis, and inflammatory myopathy.
